# FASCICULE DE BREVET EUROPEEN

(11) **EP 3 600 658 B1**
(45) Date de publication et mention de la délivrance du brevet: **20.11.2024**
(21) Numéro de dépôt: 18726058.3
(22) Date de dépôt: 30.03.2018
(51) Int. Cl.: B01J 35/00, C07C 9/00, C07C 1/00, C07C 2/00, C07C 45/00, C22B 3/00, B01J 37/03, B01J 37/06, B01J 37/12, B01J 23/889, B01J 23/00, B01J 23/02, B01J 23/34, C02F 1/64, C02F 1/72, C22B 3/20, B09C 1/00, C02F 1/52, C02F 1/66

(54) **TRAITEMENT D'EFFLUENTS LIQUIDES DE CARRIERES**
BEHANDLUNG VON ABWASSER AUS STEINBRÜCHEN
TREATING QUARRY LIQUID EFFLUENTS

(30) Priorité: 31.03.2017 FR 1752822
(43) Date de publication de la demande: 05.02.2020
(73) Titulaire: Centre National de la Recherche Scientifique, 75016 Paris (FR)
(72) Inventeur: GRISON, Claude, 34790 Grabels (FR)
(74) Mandataire: Brevalex
(86) Numéro de dépôt international: PCT/EP2018/058358
(87) Numéro de publication internationale: WO 2018/178371

(56) Documents cités:
- EP-A1- 3 305 405
- BE-A- 573 030
- US-A1- 2004 094 484
- US-A1- 2011 163 042
- US-A1- 2014 124 453
- FENG D ET AL: "TREATMENT OF ACID MINE WATER BY USE OF HEAVY METAL PRECIPITATION AND ION EXCHANGE", MINERALS ENGINEERING, PERGAMON PRESS , OXFORD, GB, vol. 13, no. 6, 1 January 2000 (2000-01-01), pages 623 - 642, XP002406563, ISSN: 0892-6875, DOI: 10.1016/S0892-6875(00)00045-5

## Description

La présente invention concerne un procédé de préparation d'un matériau solide comprenant du manganèse ainsi qu'un procédé de dépollution d'un effluent comprenant du manganèse via la mise en contact dudit effluent avec un agent oxydant le manganèse.

La présente invention concerne également un procédé de mise en oeuvre d'une réaction de synthèse organique comprenant l'utilisation d'un matériau comprenant du manganèse à titre de catalyseur.

Les carrières de pyrite sont nombreuses en Europe et en France, notamment en Normandie et en Bretagne et sont exploitées notamment pour la préparation de matériaux de construction. Le ruissellement des eaux de pluie dans ces carrières entraine l'oxydation de la pyrite qui est principalement composée de disulfures de fer. Cette transformation génère des eaux très acides, à un pH voisin de 2,5-3, et caractérisées par de fortes concentrations en sulfate de fer (typiquement comprises entre 5 et 45 mg/L). Du sulfate de manganèse est également présent dans ces eaux acides, à une concentration comprise entre 5 et 50 mg/L. Afin de répondre aux normes de rejet, les responsables industriels traitent actuellement les effluents à la soude ou à la chaux. Ce traitement est peu satisfaisant puisqu'il génère un nouveau déchet, des boues basiques chargées en hydroxydes de fer et de manganèse qui sont stockées sur sites.

La demande de brevet EP 3305405 décrit un procédé de préparation d'oxydes mixtes de manganèse, comprenant l'ajout d'hydroxyde de sodium à une solution d'un sel de manganèse.

On connait aussi un procédé de traitement des effluents miniers acides. Plus particulièrement, le procédé comprend une étape d'oxydation en utilisant du peroxyde d'hydrogène, et l'ajout d'hydroxyde de calcium, d'oxyde de fer et de sulfure de sodium (FENG D. et al., Treatment of acid mine water by use of heavy métal précipitation and ion exchange, Minerals Engineering, 1er janvier 2000, vol. 13, no 6, pages 623-642, ISSN 0892-6875).

Il existe cependant aujourd'hui encore un besoin de fournir un procédé permettant de valoriser un eflluent comprenant des métaux, de préférence tout en évitant la formation de boues basiques. Notamment, il existe un besoin de dépolluer les effluents des carrières de pyrite par un traitement contrôlé, de manière à respecter les normes de rejet imposées aux industriels.

Un objectif de la présente invention est donc de fournir un procédé permettant de dépolluer efficacement des effluents comprenant des métaux.

Un autre objectif de la présente invention est de fournir un solide comprenant au moins du manganèse et pouvant être transformé en oxyde de manganèse, et utilisé comme catalyseur de réactions d'oxydation (oxydations d'alcools, époxydation, coupure oxydante).

La présente invention concerne un procédé P1 de préparation d'un matériau solide comprenant du manganèse, ledit procédé comprenant les étapes suivantes :
a. l'ajout d'un agent oxydant le manganèse à un effluent aqueux comprenant du manganèse et comprenant en outre un ou plusieurs des éléments choisis parmi de l'aluminium, du calcium, du cuivre, du fer, du potassium, du magnésium, du sodium, du zinc, du nickel, de l'arsenic et du silicium, par exemple au moins 5 mg/L, typiquement au moins de 5 à 50 mg/L, de préférence de 7 à 25 mg/L de manganèse, de préférence à une température comprise entre 10°C et 50°C, et l'agitation du mélange de l'effluent et de l'agent oxydant pendant une durée comprise entre 1 minute et 5 heures ;
b. l'ajout à la solution obtenue à l'issue de l'étape a) d'une base jusqu'à l'obtention d'un pH compris entre 9 et 12 de préférence allant de 9 à 10,5, et l'obtention d'une solution comprenant un précipité ;
c. la filtration de la solution obtenue à l'issue de l'étape b) ; et
d. l'obtention d'un matériau solide comprenant du manganèse, et notamment du manganèse (IV) et/ou Mn (III).

Au sens de la présente invention, par « effluent », on entend un milieu liquide aqueux, pouvant être par exemple choisi parmi les effluents d'origine extractive ou industrielle par exemple issu des mines, des carrières ou de l'industrie chimique, telle que la sidérurgie et comportant des éléments métalliques. L'effluent peut également être issu du drainage rocheux acide, d'un procédé de lixiviation ou d'un procédé de préparation de catalyseur métallique. Avantageusement, l'effluent est un effluent de carrière de pyrite.

Outre le manganèse, l'effluent comprend au moins un métal choisi de préférence parmi aluminium, calcium, cuivre, fer, potassium, magnésium et sodium, typiquement ces métaux sont sous leur forme oxydée. Avantageusement, l'effluent comprend de 5 à 50 ppm de manganèse, de 2 à 180 ppm d'aluminium, de 30 à 300 ppm de calcium, de 0 à 15 ppm de fer, de 2 à 20 ppm de potassium, de 30 à 250 ppm de magnésium, de 10 à 40 ppm de sodium.

La teneur en métaux compris dans l'effluent peut être mesurée par spectrométrie d'émission atomique à plasma micro-ondes (MP-AES - Microwave Plasma - Atomic Emission Spectroscopy).

Selon la présente invention, on entend par « mise en contact » une étape d"ajout de l'agent oxydant dans l'effluent. Avantageusement, l'étape a) est menée à une température ambiante.

Avantageusement l'agent oxydant est choisi parmi le peroxyde d'hydrogène, le dioxygène ou le percarbonate de sodium, de préférence l'agent oxydant est le peroxyde d'hydrogène (H₂O₂). H₂O₂ est un agent oxydant avantageux puisqu'il est considéré comme étant écologique (« vert »), propre, peu cher, facilement disponible et non dangereux aux concentrations habituelles d'utilisation. De préférence, l'agent oxydant est ajouté en une concentration comprise entre 0,015 mL/L et 2 mUL.

De préférence, la durée de l'étape a) est comprise pendant 1 min et 1 heure, avantageusement pendant 30 min. Typiquement, l'étape a) est mise en oeuvre à température ambiante.

De préférence, dans l'étape b), la base est choisie parmi l'hydroxyde de potassium, l'hydroxyde de sodium, le carbonate de calcium, le carbonate de sodium, ou l'hydroxyde de calcium, de préférence la base est l'hydroxyde de sodium. Avantageusement, lors de l'étape b), la base est ajoutée jusqu'à l'obtention d'un pH au moins égal à 9,5, de préférence égal à 9,5.

L'étape c) peut être mise en oeuvre par tout moyen connu de l'homme du métier, par exemple par centrifugation. Outre la filtration, l'étape c) peut comprendre le lavage du matériau solide filtré. Ce solide peut être lavé à l'eau et/ou à l'éthanol. Une fois le solide lavé, il peut être séché, par exemple pendant 24 heures à une température égale à 140°C.

Avantageusement, le matériau solide obtenu à l'issue de l'étape d) comprend du manganèse, typiquement de 1 à 50 % en poids, de préférence de 1 à 20% en poids, plus préférentiellement de 2,5 à 13% en poids de manganèse. Outre le manganèse, le matériau solide obtenu à l'issue de l'étape d) peut comprendre un métal choisi parmi aluminium, calcium, cuivre, fer, potassium, magnésium, sodium, zinc, nickel, arsenic et silicium, de préférence parmi aluminium, calcium, cuivre, fer, potassium, magnésium et sodium. Avantageusement, matériau solide obtenu à l'issue de l'étape d) comprend de 5 à 15% en poids de manganèse, de 1 à 11% en poids d'aluminium, de 1 à 7 % en poids de calcium, de 0 à 5% en poids de fer, de 0 à 1% en poids de potassium, de 3 à 15% en poids ppm de magnésium, et de 0 à 1% en poids de sodium.

Avantageusement, le matériau solide obtenu à l'issue de l'étape d) du procédé P1 de l'invention comprend des oxydes de manganèse.

La présente invention concerne également un procédé P2 de dépollution d'un effluent aqueux comprenant du manganèse et comprenant en outre un ou plusieurs des éléments choisis parmi de l'aluminium, du calcium, du cuivre, du fer, du potassium, du magnésium, du sodium, du zinc, du nickel, de l'arsenic et du silicium, par exemple au moins 5 mg/L, typiquement au moins de 5 à 50 mg/L, de préférence de 7 à 25 mg/L de manganèse, et comprenant les étapes suivantes :
a. l'ajout d'un agent oxydant le manganèse à l'effluent aqueux, de préférence à une température comprise entre 10°C et 50°C, et l'agitation du mélange de l'effluent et de l'agent oxydant pendant une durée comprise entre 1 min et 5 heures ;
b. l'ajout à la solution obtenue à l'issue de l'étape a) d'une base jusqu'à l'obtention d'un pH compris entre 9 et 12 de préférence allant de 9 à 10,5, et l'obtention d'une solution comprenant un précipité ;
c. la filtration de la solution obtenue à l'issue de l'étape b) ; et
d. l'obtention d'un effluent aqueux comprenant moins de 1 ppm, de préférence moins de 0,4 ppm en manganèse, et d'un matériau solide comprenant du manganèse.

L'ensemble des modes de réalisation, variantes, et caractéristiques préférentielles du procédé P1 s'applique, seul ou selon l'une quelconque de leurs combinaisons, également au procédé P2 de dépollution de l'invention.

Avantageusement, le procédé P2 de l'invention est applicable au niveau industriel et respecte les normes industrielles de rejet imposées par la réglementation européenne. De préférence, l'effluent aqueux obtenu à l'issue de l'étape d) comprend de 0 à 1 ppm en manganèse, de 0 à 5 ppm d'aluminium, de 40 à 250 ppm de calcium, de 0 à 2 ppm de fer, de 3 à 10 ppm de potassium, de 1 à 60 ppm de magnésium, de 10 à 160 ppm de sodium.

Typiquement, les procédés de l'invention permettent d'éviter la formation de boues industrielles, et de considérer que les effluents issus des carrières de pyrite non pas comme des déchets mais comme des milieux réactionnels générateurs de catalyseurs oxydants verts.

La présente invention concerne également un matériau solide comprenant du manganèse, et notamment du manganèse (IV) et/ou Mn (III), susceptible d'être obtenu par les procédés P1 ou P2.

Avantageusement, le manganèse est intégré au sein d'une matrice minérale principalement composée d'hydroxyde de calcium, d'hydroxyde de magnésium, de sulfate de calcium, d'hydroxyde d'aluminium et de carbonate de calcium. Cette observation a été faite grâce à des analyses MP-AES et IR. En outre, des analyses XPS ont permis d'affirmer la présence de différents oxydes de Mn (IV) et Mn (III). Selon une variante, Mn₂O₃ est présent dans la matrice minérale. Des études de morphologie réalisée au microscope électronique par transmission à haute résolution montrent qu'il ne s'agit d'un simple dioxyde de manganèse, mais d'un matériau original.

Le matériau solide selon l'invention est avantageusement utilisé en tant que réactif ou catalyseur utile en chimie verte.

Il a été découvert par les inventeurs une analogie entre la structure des boues générées, c'est-à-dire un mélange basique de calcium et manganèse, et la structure du cluster Mn₄CaO₅. Ce cluster correspond au centre métallique du complexe d'oxydation de l'eau (OEC), site actif du photosystème Il (PS II), une oxydoréductase qui catalyse la photo-oxydation de l'eau chez les plantes. Ce cluster naturel a des propriétés d'oxydation de l'eau très intéressantes.

La présente invention concerne également un procédé P3 de mise en oeuvre d'une réaction de synthèse organique comprenant les étapes suivantes :
i) la préparation d'un composé comprenant du manganèse selon l'un quelconque des procédés P1 et P2 de l'invention ;
ii) la mise en oeuvre d'une réaction de synthèse organique par la mise en contact du composé obtenu à l'issue de l'étape i) à titre de catalyseur avec un milieu réactionnel.

L'ensemble des modes de réalisation, variantes, et caractéristiques préférentielles des procédés P1 et P2 s'applique, seul ou selon l'une quelconque de leurs combinaisons, également au procédé P3.

De manière surprenante, il s'est avéré que les composés préparés via les procédés P1 ou P2 de l'invention possèdent un pouvoir oxydant meilleur que celui des catalyseurs synthétisés jusqu'à présent et connus de l'homme du métier.

Avantageusement, dans le procédé P3 de l'invention, la réaction de synthèse organique est choisie parmi
- les réactions d'oxydation, de préférence parmi les réactions d'oxydation d'alcools en aldéhydes ou cétones, d'alcools en alpha d'un cycle aromatique y compris hétérocyclique, en alpha d'une double liaison, alcools aliphatiques, par exemple l'oxydation de l'alcool benzylique en benzaldéhyde et l'oxydation sélective de l'hydroxyméthyl-furfural en diformyl-furane ;
- les réactions de coupure oxydantes, de préférence les réactions de coupure oxydante de diols, d'alpha hydroxy acides, de dérivés carbonylés alpha hydroxylés, de dérivés dicarbonylés ; et
- les réactions d'époxydation d'alcènes, de préférence parmi les réactions réaction d'époxydation d'alcènes mono, di-, tri ou tétrasubstitués.

De préférence, dans le procédé P3 selon l'invention, la réaction de synthèse organique est réalisée en présence d'un agent oxydant le catalyseur, comme par exemple le dioxygène de l'air.

L'invention va maintenant être décrite grâce aux exemples suivants, non limitatifs.

### DESCRIPTION DES FIGURES

La Figure 1 montre la composition des effluents en fonction du pH de précipitation des métaux établie par MP AES
La Figure 2 montre la composition des catalyseurs en fonction du pH de précipitation établie par MP AES

### EXEMPLES

### Exemple 1 : Analyse d'effluents provenant des carrières de pyrite.

**Tableau 1 : Analyses MP-AES de différents effluents provenant de carrières de pyrite (valeurs en ppm)**

| | Al | Ca | Cu | Fe | K | Mg | **Mn** | Na |
|---|---|---|---|---|---|---|---|---|
| Effluent 1 | 5,2 | 71,4 | 0,4 | 5,1 | 6,6 | 63,2 | **13,4** | 31,8 |
| Effluent 2 | 4,3 | 65,3 | 0,5 | 1,8 | 6,3 | 58,2 | **12,3** | 31,8 |
| Effluent 3 | 3 | 55,2 | 0,7 | 2,2 | 5,3 | 36,9 | **9,1** | 26,4 |
| Effluent 4 | 56,8 | 243,5 | 0,05 | 6,4 | 5,3 | 153,2 | **14,1** | 20,8 |
| Effluent 5 | 154 | 280 | <1 | 3,4 | 5,0 | 227 | **25,2** | 24,0 |

Les effluents proviennent de carrières de pyrite différentes situées en Bretagne et en Normandie. L'effluent testé dans les exemples ci-dessous est l'effluent 3.

Ces analyses montrent que les effluents testés sont caractérisés par une teneur élevée en manganèse.

### Exemple 2 : Préparation de composés à partir d'effluents.

L'effluent 3 (850µL) a été mis sous agitation en présence de H₂O₂ (30 %, 70 eq) à température ambiante. Après 30 min, NaOH (2 M) a été ajouté goutte-à-goutte jusqu'à atteindre le pH souhaité. Un précipité noir puis jaune est apparu progressivement au furet-à-mesure de l'ajout de NaOH. La solution a été agitée à température ambiante pendant 1 nuit. Le solide précipité a été filtré et lavé à l'eau déminéralisée (3 fois) puis à l'éthanol absolu (3 fois). Le solide obtenu, noir (pH ~ 9,5), marron foncé (pH ~ 10,5) ou marron clair (pH > 11), a alors été séché à 140°C pendant 24 h.

Les teneurs en métaux ont été mesurées par MP-AES et sont présentées dans le tableau suivant.

**Tableau 2 : Analyse MP-AES des Eco-PS2 formés à différents pH de précipitation.**

| **Composé** | **pH de précipitation** | **Mn (pds %)** | **Fe (pds %)** | **Ca (pds %)** | **Mg (pds %)** | **Na (pds %)** | **Al (pds %)** | **K (pds %)** |
|---|---|---|---|---|---|---|---|---|
| **1** | 9,5 | 12,6 | 4,8 | 2,1 | 2,7 | 0,1 | 4,3 | 0, |
| **2** | 11 | 3,7 | 1,1 | 6,9 | 13,2 | 0,1 | 1,15 | 0,1 |
| **3 (comparatif)** | 14 | 2,8 | 0,3 | 14,4 | 13,2 | 0 | 1,0 | 0 |

Le pH de précipitation (étape b) influe fortement sur la teneur en métaux dans le solide final obtenu après filtration.

Une analyse spectroscopie (microscopie électronique en transmission) a montré que les composés 1 et 2 présentent une structure comprenant des « étoiles fripées » sans bâtonnets, alors que les composés commerciaux tels que MnO2 présentent une structure en bâtonnets.

En outre, les analyses XPS permettent de penser que les oxydes de manganèse (IV) présents dans les composés 1 et 2 sont associés à oxydes de manganèse (III). La présence de Mn2O3 semble être plus plausible que celle de la manganite (γ-MnOOH). Les spectres XPS des composés 1 et 2 présentent un pic Mn 2p_{3/2} à 642,4 eV et un pic Mn 2p₁ₗ₂ à 654,4 eV. Les analyses XPS ont été réalisées via un spectrophotomètre ESCALAB 250 (Thermo Electron Corporation), équipé d'une source monochromatique Al Kα X-ray (1486.6 eV).

Les analyses DRX montrent que seul le sulfate de calcium est cristallin, et les oxydes de Mn sont amorphes, comme la forme la plus active de MnO₂. Les analyses DRX ont été réalisées via un diffractomètre BRUKER (D8 advance, avec une radiation CuKα λ=1.54086°A) équipé d'un détecteur LynxEye.

Enfin, les analyses BET montrent que le composé 1 est caractérisé par une surface spécifique égale à 319m2/g et un diamètre de pores moyen égal à 130 Å, et le composé 2 est caractérisé par une surface spécifique égale à 154m2/g et un diamètre de pores moyen égal à 130 Å. Enfin, les analyses BET (Brunauer-Emmett-Teller method sont établies comme suit : le volume des pores et la moyenne des tailles de pores sont estimées à partir de la méthode Barrett-Joyner-Helenda avec l'équation Kruk-Jaroniec-Sayari équation (BJH/KJS).

L'ajout de soude dans les effluents a pour but non seulement de neutraliser l'acidité des eaux mais aussi de faire précipiter l'ensemble des métaux présents. Néanmoins, un excès de NaOH (jusqu'à pH = 14). Lorsque le pH de précipitation est égal à 14, la quasi-totalité du calcium et du magnésium contenus dans les effluents précipite et les catalyseurs formés sont alors majoritairement composés des hydroxydes correspondants. Ces derniers peuvent donc moduler l'activité du composé, mais aussi retenir les réactifs et/ou les produits à la surface de la matrice minérale du composé.

Si le calcium peut avoir un effet activant sur le manganèse, au contraire, le magnésium est décrit dans la littérature comme ayant un effet antagoniste. Il est donc préférable de contrôler le pH de précipitation afin de faire précipiter sélectivement les métaux.

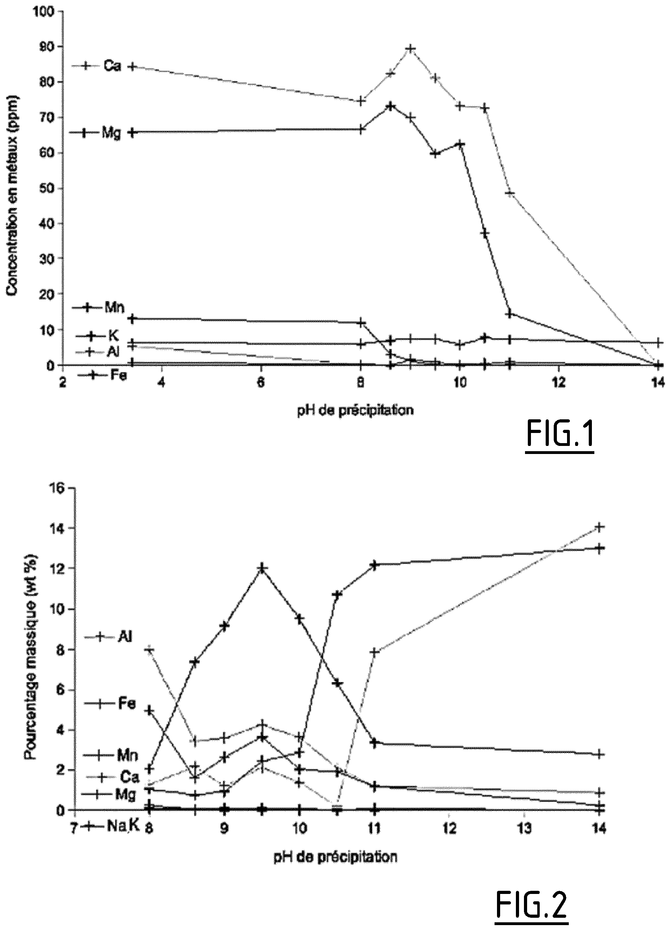

Les analyses MP-AES présentées sur la Figure 1 indiquent que la concentration en manganèse dans les effluents après précipitation des métaux est inférieure à 1,8 ppm dès que le pH est égal à 9.

De ce fait, afin de respecter les normes industrielles imposées, il est préférable que le pH de précipitation soit supérieur à 9. Ensuite, les analyses MP-AES (Figure 2) des solides formés en faisant varier le pH de précipitation montrent que la plus forte teneur en manganèse est obtenue pour un pH d'environ 9,5, atteignant environ 13 wt %. De plus, il convient de noter que, dans ce cas, le rapport molaire Mn/Ca est très proche de celui du cluster naturel (Mn₄,Ca) dérivé du photosystème 2.

### Exemple 3 : Réaction de synthèse organique

Pour tester l'activité des matériaux de l'invention, l'oxydation de l'alcool benzylique en benzaldéhyde a été prise comme réaction modèle.

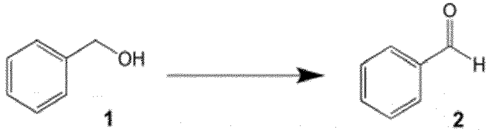

Le mode opératoire adopté a été le suivant : le réactif (100 mmol.L⁻¹) a été mis en présence du solide préparé dans l'exemple 2 dans le toluène anhydre et la solution a été chauffée à 110°C pendant 5 h. Le mélange réactionnel a ensuite été analysé par GC-MS afin de déterminer la conversion et la sélectivité en utilisant le dodécane comme étalon interne.

Les résultats obtenus sont présentés dans le Tableau suivant (le pH correspond au pH obtenu lors de la précipitation du composé).

**Tableau 3. Comparaison du pH de précipitation sur l'activité catalytique oxydante des solides lors de l'oxydation de l'alcool benzylique en benzaldéhyde.**

| Entrée | pH | Eq. Mn | Conversion ^{b} (%) | Rendement ^{b} (%) | Sélectivité ^{b,c} (%) |
|---|---|---|---|---|---|
| 1 | 9,5 | 1 | 70 | 69 | >99 |
| 2^{d} | 9,5 | 0,3 | 22 | 21 | >99 |
| 3 | 10 | 1 | <93 ^{e} | >54 ^{e} | - |
| 4^{d} | 10 | 0,1 | 8 | 7 | >99 |
| 5^{d} | 10,5 | 0,3 | 73 | 72 | >99 |
| 6 | 11 | 1, | <86 ^{e} | >44^{e} | - |
| 7^{d} | 11 | 0,3 | 79 | 78 | >99 |
| 8 (comparatif) | 14 | 1 | <45 ^{e} | >16 ^{e} | - |
| 9^{d} (comparatif) | 14 | 0,3 | 10 | 9 | >99 ^{e} |

| | | | | | |
|---|---|---|---|---|---|
| *a Conditions réactionnelles : alcool benzylique 1 (100 mmol.L-1), catalyseur, toluène anhydre, reflux à 110°C, 5 h. b La conversion, le rendement et la sélectivité ont été déterminés par GC-MS, en utilisant le dodécane comme étalon interne. c Ratio du rendement GC-MS en aldéhyde sur la conversion, d Réaction réalisée avec bullage d'air. e Perte de réactifs sur la matrice du catalyseur.* | | | | | |

Les premières réactions engageant les composés préparés via le procédé de l'invention (pH = 9,5) et l'alcool benzylique en quantité stoechiométrique montrent un rendement en benzaldéhyde de 70 % avec une excellente sélectivité après 5 h de réaction. De plus, aucune perte en réactif ni en produit par rétention sur la matrice minérale du composé n'est constatée, contrairement à ce qui est observé pour les composés formés à des pH plus élevés.

Cependant, lorsque les composés préparés via le procédé de l'invention (pH = 9,5) sont utilisés en quantité catalytique (0,3 eq) avec bullage d'air pour ré-oxyder le catalyseur grâce au dioxygène, la réaction ne se produit qu'en quantité stoechiométrique (conversion de 20 %). Le dioxygène contenu dans l'air ne permet pas de ré-oxyder les solides préparés à pH = 9,5, 10 et 14.

Au contraire, une ré-oxydation des composés (pH = 10,5) et solides (pH = 11) par le dioxygène de l'air est observée. Lorsque les composés sont engagés en quantité catalytique (0,3 eq), une conversion de 73 % et 79 % respectivement est obtenue en 5 h de réaction avec une sélectivité totale en benzaldéhyde. 100 % de rendement est obtenu après 7 h de réaction.

Par conséquent, la modification du pH de précipitation permet la synthèse de composés à base d'oxyde de manganèse au pouvoir oxydant bien supérieur à celui observé avec des composés préparés avec un pH de précipitation égale à 14. Les composés préparés via le procédé de l'invention montrent une excellente sélectivité en benzaldéhyde, sans sur-oxydation en acide benzoïque. Les composés préparés à pH = 11 et pH = 10,5 ont la particularité de pouvoir être ré-oxydés par l'oxygène de l'air.

### Exemple 4 : Comparaison de l'activité avec des catalyseurs synthétiques et commerciaux

Afin de déterminer l'origine de l'activité des composés préparés via le procédé de l'invention, des catalyseurs synthétiques ont été préparés. Étant donné que le pH de précipitation influe sur l'activité et la ré-oxydation des composés grâce au dioxygène de l'air, il est attendu que le calcium et/ou le magnésium, principaux éléments affectés par la modification du pH dans la gamme 9-12, jouent un rôle dans l'activité des catalyseurs formés.

Différents catalyseurs synthétiques ont été préparés à partir de sels de manganèse, calcium et magnésium.

La préparation de ces catalyseurs a été identique à celle suivie pour synthétiser les solides de l'invention préparés à un pH = 11 à partir de l'effluent 3. Les catalyseurs synthétiques dérivent de produits commerciaux MnSO₄, CaSO₄, MgSO₄. Les catalyseurs 3, 4 et 5 du tableau 4 sont reconstitués de façon à respecter les ratio en Mn, Mg et Ca du solide de l'invention issu de l'effluent 3.

Les concentrations en sels engagées sont identiques à celles des effluents, sauf dans le cas des catalyseurs Mn-synthétiques où la concentration en MnSO₄.H₂O a été multipliée par 4 par rapport à la concentration en sulfate de manganèse dans les effluents pour obtenir davantage de matériel sur lequel travailler.

L'activité catalytique de ces catalyseurs a été testée dans les mêmes conditions que pour les composés de l'invention, en prenant l'oxydation de l'alcool benzylique en benzaldéhyde comme réaction modèle. Les résultats sont présentés dans le Tableau 4.

**Tableau 4. Comparaison de l'activité catalytique oxydante des solides de l'invention (pH = 11) avec celle de catalyseurs synthétiques et commerciaux lors de l'oxydation de l'alcool benzylique en benzaldéhyde.**

| **Entrée** | **Catalyseur** | **Eq. Mn** | **Conversion ^{b} (%)** | **Rendement ^{b} (%)** | **Sélectivité (%)** |
|---|---|---|---|---|---|
| **1** | Solide selon l'invention préparé à pH = 11 | 0,3 | 79% | 78% | >99% |
| **2** | Mn-synthétique | 0,3 | 100% | 99% | >99% |
| **3** | MnCa-synthétique | 0,3 | 100% | 99% | >99% |
| **4** | MnMg-synthétique | 0,3 | 67% | 66% | >99% |
| **5** | MnCaMgsynthétique | 0,3 | 90% | 89% | >99% |
| **6** | MnO₂ activé commercial^{d} | 1 | 89% | 88% | >99% |
| **7** | MnO₂ activé commercial | 0,3 | 38% | 37% | >99% |

| | | | | | |
|---|---|---|---|---|---|
| a *Conditions réactionnelles : alcool benzylique 1 (100 mmol.L-1), catalyseur, toluène anhydre, bullage d'air, reflux à 110°C,5 h. b La conversion, le rendement et la sélectivité ont été déterminés par GC-MS, en utilisant le dodécane comme étalon interne. c Ratio du rendement GC-MS en aldéhyde sur la conversion, d Réaction réalisée sans bullage d'air.* | | | | | |

La conversion en benzaldéhyde est de 100 % dans les cas où le manganèse n'est couplé à aucun autre métal ainsi qu'en présence de calcium (Tableau 4, entrées 2-3). Le procédé de l'invention permet donc d'obtenir un oxyde de manganèse (IV) activé, plus actif que MnO₂, y compris MnO₂ activé. Ces résultats ne permettent pas de savoir si la présence de calcium au sein du catalyseur à un effet positif ou neutre sur sa réactivité. Au contraire, la présence de magnésium semble réduire l'activité du catalyseur, puisque le rendement GC-MS diminue à 66 % et 89 % pour les catalyseurs MnMg-synthétique et MnCaMg-synthétique respectivement.

Par comparaison, la réaction d'oxydation de l'alcool benzylique a également été testée dans les mêmes conditions avec du MnO₂ activé commercial. Introduit en quantité stoechiométrique, la réactivité est similaire à celle des composés de l'invention, avec un rendement de 88 % en benzaldéhyde (Tableau 4, entrée 6). Cependant, lorsqu'il est introduit en quantité catalytique, le MnO₂ activé commercial n'est pas (ou très peu) ré-oxydé par le dioxygène de l'air, étant donné que le rendement n'est que de 37 % (Tableau 4, entrée 7). Ces résultats sont en accord avec les données de la littérature, à savoir que le MnO₂ activé commercial se doit d'être introduit en excès pour réaliser l'oxydation des substrats organiques.

En conclusion, vis-à-vis de l'oxydation de l'alcool benzylique en benzaldéhyde, les composés de l'invention (préparés à pH = 11) ont une activité catalytique oxydante supérieure à celle de MnO₂ activé commercial. Cette réactivité semble intrinsèque au mode opératoire de synthèse mis en place, étant donné que les catalyseurs Mn-synthétiques montrent une activité supérieure à celle des solides de l'invention (pH = 11). Comme attendu, le magnésium a un effet antagoniste sur la réactivité des catalyseurs mais les expériences réalisées ne permettent pas de conclure quant à l'effet de synergie entre le manganèse et le calcium. Par conséquent, le mode opératoire employé permet de former un oxyde de manganèse (IV) activé avec un fort pouvoir oxydant.

Enfin, il est important de prendre en considération l'empreinte environnementale que la synthèse des catalyseurs Mn-synthétiques implique comparée à celle des composés de l'invention. En effet, le sulfate de manganèse utilisé pour synthétiser les catalyseurs Mn-synthétiques est en général préparé par traitement de MnO₂ avec du dioxyde de soufre ou bien par réaction du permanganate de potassium avec de l'hydrogénosulfate de sodium et du peroxyde d'hydrogène. En plus des performances catalytiques, il est important de prendre en considération l'analyse du cycle de vie (ACV) des catalyseurs formés afin que le procédé de synthèse s'inscrive dans une démarche de développement durable.

### Exemple 5 : Utilisation des solides de l'invention dans l'oxydation sélective du HMF (HydroxyMéthylFurfural) en DFF (DiFormyl Furane)

Les composés de l'invention (pH = 11) ont été utilisés en tant que catalyseur oxydant dans la réaction d'oxydation sélective du HMF en DFF. 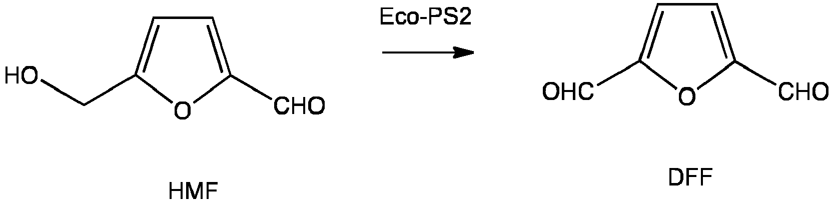 Du HMF (126 mg, 1 mmol) a été dissous dans du méthoxycyclopentane (2 mL), le catalyseur (0,3 mol eq Mn) et 10 mL de toluène anhydre ont été placés dans un tricol. La solution a été mise sous agitation et chauffée à reflux à 110°C en présence d'un bullage d'air pendant 5 h. La solution a ensuite été acidifiée par une solution aqueuse d'acide sulfurique à pH = 3,3 (10 mL). De l'acétate d'éthyle (10 mL) a été ajouté et la solution a été mise sous agitation pendant 15 min. La solution a été filtrée et le solide a été lavé trois fois avec 10 mL d'acétate d'éthyle. La phase aqueuse a été extraite avec trois fois 10 mL d'acétate d'éthyle. Les différentes phases organiques ont été rassemblées et le solvant a été évaporé. Un solide jaune orangé a été obtenu. La conversion et la sélectivité ont été déterminées par GC-MS, en utilisant le biphényle comme étalon interne. Les résultats sont présentés dans le tableau 5.

**Tableau 5. Conversion en HMF et sélectivité en DFF obtenus avec les Eco-PS2 comme catalyseurs.**

| **Entrée** | **Catalyseur** | **Eq. Mn** | **Conversion b (%)** | **Sélectivité b, c (%)** |
|---|---|---|---|---|
| **1** | Solide selon l'invention préparé à pH = 11 | 1 | 50% | 75% |
| **3** | Solide selon l'invention préparé à pH = 11 | 0,3 | 45% | 71% |
| **3** | Solide selon l'invention préparé à pH = 9,5 | 1 | 60% | 77% |

| | | | | |
|---|---|---|---|---|
| *a Conditions réactionnelles : HMF 3 (100 mmol.L-1) dissous dans CPME, catalyseur, toluène anhydre, bullage d'air, reflux à 110°C, 5 h. b La conversion, le rendement et la sélectivité ont été déterminés par GC-MS, en utilisant le biphényle comme étalon interne. c Ratio du rendement GC-MS en aldéhyde sur la conversion.* | | | | |

Les analyses GC-MS ne montrent la présence d'aucun autre produit à part HMF et DFF. L'utilisation des composés de l'invention (pH = 11) en quantité stoechiométrique ou catalytique donnent les mêmes résultats en termes de conversion et sélectivité (Tableau 5, entrée 1-2). Dans les deux cas, la conversion est proche de 50 %.

La conversion et le rendement obtenus avec les solides de l'invention (pH = 9,5) sont légèrement supérieurs à ceux obtenus avec les solides de l'invention ( pH = 11), avec 60 % de conversion (Tableau 5, entrée 3). Dans tous les cas, la sélectivité en DFF est proche de 75 %. Afin de déterminer la présence ou non d'acides carboxyliques, des analyses IR et LC MS ont confirmé la formation du HMF et du DFF.

Le N,O-bis(triméthylsilyl)trifluoroacétamide a été utilisé comme agent silylant. L'analyse GC-MS des produits de silylation n'indique la présence d'aucun autres composés que le DFF et le HMF silylé. La sélectivité de la réaction est donc très élevée et supérieure aux méthodes de la littérature qui décrivent la formation de mono et diacides.

### Exemple 6 : Réactions d'oxydation

Le procédé de l'invention a été mis en oeuvre dans plusieurs réactions d'oxydations à l'aide du catalyseur issu de l'effluent 2. Les résultats sont présentés dans le tableau 6.

**Tableau 6 : Réactions d'oxydation**

| Entrée | Alcool | Aldéhyde | Conversion (%) |
|---|---|---|---|
| 1 | 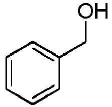 | 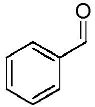 | 100 |
| 2 | 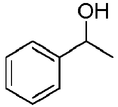 | 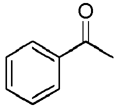 | 98 |
| 3 | 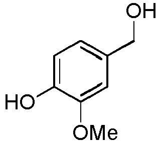 | 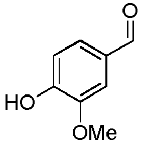 | 61 |
| 5 | 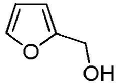 | 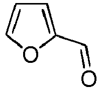 | 49 |
| 6 | 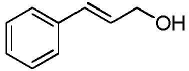 | 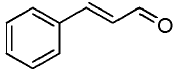 | 94 (54% de rendement) |

L'oxydation est compatible avec le groupement OH du phénol. On oxyde l'alcool primaire sans toucher le noyau phénolique (entrée 3). Cette réaction permet d'obtenir la vanilline, le produit très recherché dans l'industrie alimentaire, cosmétique, parfumerie etc. L'oxydation ne dégrade pas le noyau furanique (entrées 4 et 5). La réaction s'arrête au dialdéhyde. Aucune trace d'acide ou de diacide n'est observée, ni en GC/MS, ni après le traitement du milieu avec un acide inorganique suivi d'une extraction. Le dialdéhyde est un building-block biosourcé très intéressant (voir J. Ma, Z. Du, J. Xu, Q Chu, Y. Pang ChemSusChem, 2011, 4, 51-54 ; A. Gandini, Green Chem., 2011, 13, 1061-1083).

L'alcool cinnamique est presque complètement oxydé en aldéhyde correspondant (entrée 6). Le produit aldéhyde est isolé avec 54% de rendement et 40% de produit de condensation est obtenu comme sous-produit de réaction. L'aldéhyde cinnamique est un produit très recherché dans l'industrie alimentaire, cosmétique, parfumerie etc.

Les avantages de ce procédé par rapport aux procédés existants sont les suivants :
- la réaction se fait à la pression atmosphérique
- Il n'est pas nécessaire de faire barboter du dioxygène pur ou de faire la réaction sous pression d'O₂. Notre procédé fonctionne soit à l'air soit en faisant barboter l'air dans le milieu réactionnel.
- la quantité de manganèse utilisée dans la réaction est de 10 mol% à 50 mol% en Mn, ce qui est très inférieur aux procédés existants.

### Exemple 7 : Coupure oxydante

Le procédé peut également être étendu à la coupure oxydante des a-diols, a-hydroxycétones, a-hydroxyacides. Les résultats sont présentés dans le tableau 8.

**Tableau 8 : Coupure oxydante à l'aide du catalyseur issu de l'effluent 2**

| **Entrée** | **Alcool** | **% Benzaldehyde** | **Conversion** |
|---|---|---|---|
| 1 | 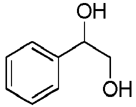 | 100% | 100% |
| 2 | 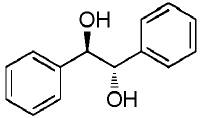 | 84% | 100% |
| 3 | 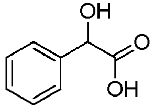 | >95 | >95% |

Les avantages de ce procédé sont les suivants :
- la préparation du catalyseur nécessite une faible quantité de soude ou autre base.
- pas de base utilisée lors de la réaction
- pas besoin de faire barboter de l'oxygène pur ou faire la réaction sous pression dans l'atmosphère d'oxygène pur. Notre procédé fonctionne soit à l'air soit en barbotant de l'air dans le milieu réactionnel
- la réaction se fait à la pression atmosphérique et avec de faibles quantités de Mn

### Exemple 8 : Epoxydation d'alcènes

L'époxydation des alcènes peut également être facilement réalisée à partir de l'effluent industriel en présence d'un co-oxydant tel que l'eau oxygénée. La méthode peut être avantageusement comparée aux méthodes de la littérature.

### Mode opératoire générale pour la réaction d'époxydation :

NaHCO₃ (0,007g ; 0,09 mol ; 5 éq), effluent 2 (0,26 mL (pH = 3,5 ; Mn = 12 ppm) ; 0,001 éq par rapport au Mn), t-BuOH ou DMF (0,263 mL) et alcène (0,02 mol ; 1 éq) à 30 °C à l'air. Après 10 minutes d'agitation, l'H₂O₂30% (0,016 mL ; 0,17 mol ; 10 éq) est ajouté au mélange réactionnel à 30 °C à l'air. Le dégagement gazeux est observé au bout d'une minute. L'agitation est maintenue encore quatre heures, puis la réaction est refroidie à température ambiante. Le produit est extrait avec du dichlorométhane et analysé en GC MS.

Les conversions sont présentées dans le tableau 8.

**Tableau 8 : Epoxydation d'alcènes**

| Substrat | Eco-PS2 / *t*BuOH | Eco-PS2/DMF | EcoMn** (DMF) | littérature |
|---|---|---|---|---|
| ***Styrène*** | 91% | - | 91% | |
| ***Cyclooctène*** | 81% | - | 55% | |
| ***Cyclohexène*** | 86% | - | 89% | |
| ***Isoeugénol*** | 0% | 50%* | Coupure oxydante | |
| ***Pinène*** | traces | 100% | 75% | 40 % (Qi, B. J. Mol. Cat. A 2010, 322, 73) |
| ***Limonène*** | traces | 92% | 43% | |
| ***Linalool*** | traces | 95% | 63% | |
| ***Nopol*** | traces | 45% | 74% | |

| | | | | |
|---|---|---|---|---|
| *Dans le cas d'isoeugénol la GCMS nous indique la formation d'une famille des produits d'autocondensation d'isoeugénol. Le produit majoritaire semble être la Licarine A. **Eco-Mn dérivés des accumulateurs de Mn du genre Grevillea | | | | |

Les solides Eco-PS2 représentent les catalyseurs préparés via le procédé de l'invention.

## Revendications

1. Procédé de préparation d'un matériau solide comprenant du manganèse, ledit procédé comprenant les étapes suivantes :
a. l'ajout d'un agent oxydant le manganèse à un effluent aqueux comprenant du manganèse et comprenant en outre un ou plusieurs des éléments choisis parmi de l'aluminium, du calcium, du cuivre, du fer, du potassium, du magnésium, du sodium, du zinc, du nickel, de l'arsenic et du silicium, par exemple au moins 5 mg/L, typiquement au moins de 5 à 50 mg/L, et de préférence de 7 à 25 mg/L de manganèse, de préférence à une température comprise entre 10°C et 50°C, et l'agitation du mélange de l'effluent et de l'agent oxydant pendant une durée comprise entre 1 minute et 5 heures ;
b. l'ajout à la solution obtenue à l'issue de l'étape a) d'une base jusqu'à l'obtention d'un pH compris entre 9 et 12, de préférence allant de 9 à 10,5, et l'obtention d'une solution comprenant un précipité ;
c. la filtration de la solution obtenue à l'issue de l'étape b) ; et
d. l'obtention d'un matériau solide comprenant du manganèse, et notamment du manganèse (IV) et/ou Mn (III).

2. Procédé selon la revendication 1, dans lequel le matériau solide obtenu à l'issue de l'étape d) comprend des oxydes de manganèse.

3. Procédé de dépollution d'un effluent aqueux comprenant du manganèse et comprenant en outre un ou plusieurs des éléments choisis parmi de l'aluminium, du calcium, du cuivre, du fer, du potassium, du magnésium, du sodium, du zinc, du nickel, de l'arsenic et du silicium, par exemple au moins 5 mg/L, typiquement au moins de 5 à 50 mg/L, de préférence de 7 à 25 mg/L de manganèse, et comprenant les étapes suivantes :
a. l'ajout d'un agent oxydant le manganèse à l'effluent aqueux, de préférence à une température comprise entre 10°C et 50°C, et l'agitation du mélange de l'effluent et de l'agent oxydant pendant une durée comprise entre 1 min et 5 heures ;
b. l'ajout à la solution obtenue à l'issue de l'étape a) d'une base jusqu'à l'obtention d'un pH compris entre 9 et 12, de préférence allant de 9 à 10,5, et l'obtention d'une solution comprenant un précipité ;
c. la filtration de la solution obtenue à l'issue de l'étape b) ; et
d. l'obtention d'un effluent aqueux comprenant moins de 1 ppm, de préférence moins de 0,4 ppm en manganèse, et d'un matériau solide comprenant du manganèse.

4. Procédé selon l'une quelconque des revendications précédentes, dans lequel l'agent oxydant est le peroxyde d'hydrogène.

5. Procédé selon l'une quelconque des revendications précédentes dans lequel la base est choisie parmi l'hydroxyde de potassium, l'hydroxyde de sodium, le carbonate de calcium, le carbonate de sodium et l'hydroxyde de calcium, de préférence la base est l'hydroxyde de sodium.

6. Procédé selon l'une quelconque des revendications précédentes dans lequel, dans l'étape b) la base est ajoutée jusqu'à l'obtention d'un pH au moins égal à 9,5, de préférence égal à 9,5.

7. Procédé selon l'une quelconque des revendications précédentes dans lequel l'effluent comprend de 5 à 50 ppm de manganèse, de 2 à 180 ppm d'aluminium, de 30 à 300 ppm de calcium, de 0 à 15 ppm de fer, de 2 à 20 ppm de potassium, de 30 à 250 ppm de magnésium, de 10 à 40 ppm de sodium.

8. Matériau solide comprenant du manganèse, et notamment du manganèse (IV) et/ou Mn (III), susceptible d'être obtenu par un procédé tel que défini selon la revendication 7.

9. Matériau selon la revendication précédente, comprenant de 5 à 15% en poids de manganèse, de 1 à 11% en poids d'aluminium, de 1 à 7 % en poids de calcium, de 0 à 5% en poids de fer, de 0 à 1% en poids de potassium, de 3 à 15% en poids ppm de magnésium, et de 0 à 1% en poids de sodium.

10. Procédé de mise en oeuvre d'une réaction de synthèse organique comprenant la mise en contact d'un matériau solide comprenant du manganèse selon la revendication 9 avec un milieu réactionnel, la réaction de synthèse organique est choisie parmi :
- les réactions d'oxydation, de préférence parmi les réactions d'oxydation d'alcools en aldéhydes ou cétones, d'alcools en alpha d'un cycle aromatique y compris hétérocyclique, en alpha d'une double liaison, alcools aliphatiques, par exemple l'oxydation de l'alcool benzylique en benzaldéhyde et l'oxydation sélective de l'hydroxyméthyl-furfural en diformyl-furane,
- les réactions de coupure oxydantes, de préférence les réactions de coupure oxydante de diols, d'alpha hydroxy acides, de dérivés carbonylés alpha hydroxylés, de dérivés dicarbonylés ; et
- les réactions d'époxydation d'alcènes, de préférence parmi les réactions réaction d'époxydation d'alcènes mono, di-, tri ou tétrasubstitués.

11. Procédé selon la revendication précédente dans lequel la réaction de synthèse organique est réalisée en présence d'un agent oxydant le catalyseur, comme par exemple le dioxygène de l'air.

## Patentansprüche

1. Verfahren zum Herstellen eines festen Materials, das Mangan umfasst, wobei das Verfahren die folgenden Schritte umfasst:
a. Zugeben eines Mangan-Oxidationsmittels zu einem wässrigen Abwasser, das Mangan umfasst und ferner eines oder mehrere der Elemente umfasst, die aus Aluminium, Calcium, Kupfer, Eisen, Kalium, Magnesium, Natrium, Zink, Nickel, Arsen und Silizium ausgewählt ist, zum Beispiel mindestens 5 mg/L, typischerweise mindestens 5 bis 50 mg/L und vorzugsweise 7 bis 25 mg/L Mangan, vorzugsweise bei einer Temperatur zwischen 10 °C und 50 °C, und Rühren der Mischung aus dem Abwasser und dem Oxidationsmittel für einen Zeitraum zwischen 1 Minute und 5 Stunden;
b. Zugeben einer Base zu der am Ende von Schritt a) erhaltenen Lösung, bis ein pH-Wert zwischen 9 und 12, vorzugsweise im Bereich von 9 bis 10,5, erreicht wird, und Erhalten einer Lösung, die eine Ausfällung umfasst;
c. Filtrieren der am Ende von Schritt b) erhaltenen Lösung; und
d. Erhalten eines festen Materials, das Mangan und insbesondere Mangan (IV) und/oder Mn (III) umfasst.

2. Verfahren nach Anspruch 1, wobei das am Ende von Schritt d) erhaltene feste Material Manganoxide umfasst.

3. Verfahren zum Reinigen eines wässrigen Abwassers, das Mangan umfasst und ferner eines oder mehrere der Elemente umfasst, die aus Aluminium, Calcium, Kupfer, Eisen, Kalium, Magnesium, Natrium, Zink, Nickel, Arsen und Silizium ausgewählt ist, zum Beispiel mindestens 5 mg/L, typischerweise mindestens 5 bis 50 mg/L, vorzugsweise 7 bis 25 mg/L Mangan, und das die folgenden Schritte umfasst:
a. Zugeben eines Mangan-Oxidationsmittels zu dem wässrigen Abwasser, vorzugsweise bei einer Temperatur zwischen 10 °C und 50 °C, und Rühren der Mischung aus dem Abwasser und dem Oxidationsmittel für einen Zeitraum zwischen 1 Minute und 5 Stunden;
b. Zugeben einer Base zu der am Ende von Schritt a) erhaltenen Lösung, bis ein pH-Wert zwischen 9 und 12, vorzugsweise im Bereich von 9 bis 10,5, erreicht wird, und Erhalten einer Lösung, die eine Ausfällung umfasst;
c. Filtrieren der am Ende von Schritt b) erhaltenen Lösung; und
d. Erhalten eines wässrigen Abwassers, das weniger als 1 ppm, vorzugsweise weniger als 0,4 ppm Mangan umfasst, und eines festen Materials, das Mangan umfasst.

4. Verfahren nach einem der vorhergehenden Ansprüche, wobei das Oxidationsmittel Wasserstoffperoxid ist.

5. Verfahren nach einem der vorhergehenden Ansprüche, wobei die Base aus Kaliumhydroxid, Natriumhydroxid, Calciumcarbonat, Natriumcarbonat und Calciumhydroxid ausgewählt ist, wobei die Base vorzugsweise Natriumhydroxid ist.

6. Verfahren nach einem der vorhergehenden Ansprüche, wobei in Schritt b) die Base zugegeben wird, bis ein pH-Wert von mindestens gleich 9,5, vorzugsweise gleich 9,5, erreicht wird.

7. Verfahren nach einem der vorhergehenden Ansprüche, wobei der Ausfluss 5 bis 50 ppm Mangan, 2 bis 180 ppm Aluminium, 30 bis 300 ppm Calcium, 0 bis 15 ppm Eisen, 2 bis 20 ppm Kalium, 30 bis 250 ppm Magnesium und 10 bis 40 ppm Natrium umfasst.

8. Festes Material, das Mangan und insbesondere Mangan (IV) und/oder Mn (III) umfasst, das durch ein gemäß Anspruch 7 definiertes Verfahren erhalten werden kann.

9. Material nach dem vorhergehenden Anspruch, das 5 bis 15 Gew.-% Mangan, 1 bis 11 Gew.-% Aluminium, 1 bis 7 Gew.-% Calcium, 0 bis 5 Gew.-% Eisen, 0 bis 1 Gew.-% Kalium, 3 bis 15 Gew.-% ppm Magnesium und 0 bis 1 Gew.-% Natrium umfasst.

10. Verfahren zum Durchführen einer organischen Synthesereaktion, umfassend das Inkontaktbringen eines festen Materials, das Mangan umfasst, nach Anspruch 9 mit einem Reaktionsmedium, wobei die organische Synthesereaktion ausgewählt ist aus:
- Oxidationsreaktionen, vorzugsweise Oxidationsreaktionen von Alkoholen zu Aldehyden oder Ketonen, von Alkoholen im alpha-Bereich eines aromatischen Rings, einschließlich heterocyclischer Ringe, im alpha-Bereich einer Doppelbindung, aliphatischer Alkohole, zum Beispiel die Oxidation von Benzylalkohol zu Benzaldehyd und die selektive Oxidation von Hydroxymethylfurfural zu Diformylfuran,
- oxidative Spaltungsreaktionen, vorzugsweise oxidative Spaltungsreaktionen von Diolen, alpha-Hydroxysäuren, alpha-Hydroxy-Carbonylderivaten und Dicarbonylderivaten; und
- Epoxidationsreaktionen von Alkenen, vorzugsweise unter den Epoxidationsreaktionen von mono-, di-, tri- oder tetrasubstituierten Alkenen.

11. Verfahren nach dem vorhergehenden Anspruch, wobei die organische Synthesereaktion in Gegenwart eines den Katalysator oxidierenden Mittels, wie zum Beispiel Sauerstoff aus der Luft, durchgeführt wird.

## Claims

1. A method for preparing a solid material comprising manganese, said method comprising the following steps:
a. adding a manganese oxidising agent to an aqueous effluent comprising manganese and further comprising one or more of the elements selected from aluminium, calcium, copper, iron, potassium, magnesium, sodium, zinc, nickel, arsenic and silicon, for example at least 5 mg/L, typically at least 5 to 50 mg/L, and preferably 7 to 25 mg/L of manganese, preferably at a temperature comprised between 10°C and 50°C, and stirring the mixture of the effluent and the oxidising agent for a duration comprised between 1 minute and 5 hours;
b. adding a base to the solution obtained at the end of step a) until a pH comprised between 9 and 12, preferably ranging from 9 to 10.5, and a solution comprising a precipitate is obtained;
c. filtering the solution obtained at the end of step b); and
d. obtaining a solid material comprising manganese, and in particular manganese (IV) and/or Mn (III).

2. The method according to claim 1, wherein the solid material obtained at the end of step d) comprises manganese oxides.

3. The method for decontaminating an aqueous effluent comprising manganese and further comprising one or more of the elements selected from aluminium, calcium, copper, iron, potassium, magnesium, sodium, zinc, nickel, arsenic and silicon, for example at least 5 mg/L, typically at least 5 to 50 mg/L, preferably 7 to 25 mg/L of manganese, and comprising the following steps:
a. adding a manganese oxidising agent to the aqueous effluent, preferably at a temperature comprised between 10°C and 50°C, and stirring the mixture of effluent and oxidising agent for a duration comprised between 1 min and 5 hours;
b. adding a base to the solution obtained at the end of step a) until a pH comprised between 9 and 12, preferably ranging from 9 to 10.5 is obtained, and obtaining a solution comprising a precipitate;
c. filtering the solution obtained at the end of step b); and
d. obtaining an aqueous effluent comprising less than 1 ppm, preferably less than 0.4 ppm of manganese, and a solid material comprising manganese.

4. The method according to any one of the preceding claims, wherein the oxidising agent is hydrogen peroxide.

5. The method according to any one of the preceding claims wherein the base is selected from potassium hydroxide, sodium hydroxide, calcium carbonate, sodium carbonate and calcium hydroxide, preferably the base is sodium hydroxide.

6. The method according to any one of the preceding claims, wherein, in step b), the base is added until a pH of at least 9.5, preferably equal to 9.5, is obtained.

7. The method according to any one of the preceding claims wherein the effluent comprises from 5 to 50 ppm manganese, from 2 to 180 ppm aluminium, from 30 to 300 ppm calcium, from 0 to 15 ppm iron, from 2 to 20 ppm potassium, from 30 to 250 ppm magnesium, from 10 to 40 ppm sodium.

8. A solid material comprising manganese, and in particular manganese (IV) and/or Mn (III), capable of being obtained by a method as defined according to claim 7.

9. The material according to the preceding claim, comprising from 5 to 15% by weight of manganese, from 1 to 11% by weight of aluminium, from 1 to 7% by weight of calcium, from 0 to 5% by weight of iron, from 0 to 1% by weight of potassium, from 3 to 15% by weight ppm of magnesium, and from 0 to 1% by weight of sodium.

10. A method for carrying out an organic synthesis reaction comprising contacting a solid material comprising manganese according to claim 9 with a reaction medium, the organic synthesis reaction is selected from:
- oxidation reactions, preferably among the reactions of oxidation of alcohols into aldehydes or ketones, of alcohols into the alpha of an aromatic ring including heterocyclic, into the alpha of a double bond, aliphatic alcohols, for example the oxidation of benzyl alcohol into benzaldehyde and the selective oxidation of hydroxymethyl-furfural into diformyl-furan,
- oxidative cleavage reactions, preferably oxidative cleavage reactions of diols, alpha hydroxy acids, alpha hydroxy carbonyl derivatives, dicarbonyl derivatives; and
- epoxidation reactions of alkenes, preferably among the epoxidation reactions of mono-, di-, tri- or tetrasubstituted alkenes.

11. The method according to the preceding claim wherein the organic synthesis reaction is carried out in the presence of an agent oxidising the catalyst, such as for example atmospheric oxygen.
